# EUROPÄISCHE PATENTSCHRIFT

(11) **EP 1 362 924 B1**
(45) Veröffentlichungstag und Bekanntmachung des Hinweises auf die Patenterteilung: **11.07.2007**
(21) Anmeldenummer: 03010508.4
(22) Anmeldetag: 09.05.2003
(51) Int. Cl.: C12M 1/107

(54) **Verfahren zur Inbetriebnahme von methanhaltige Gase erzeugenden oder führenden Einrichtungen, insbesondere Biogasanlagen oder biogasführende Leitungen**
Process for running methane-producing or methane-containing facilities, in particular biogas facilities or biogas-containing tubes
Procédé pour actionner les équipements méthane-producteurs ou méthane-contenants, en particulier les équipements de biogas ou les tubes contenant le biogas

(30) Priorität: 10.05.2002 DE 10220675
(43) Veröffentlichungstag der Anmeldung: 19.11.2003
(73) Patentinhaber: LINDE-KCA-Dresden GmbH, 01277 Dresden (DE)
(72) Erfinder: Hiecke, Jörg, 01097 Dresden (DE)

(56) Entgegenhaltungen:
- DD-A- 228 535
- DE-A- 3 737 870
- DE-A- 10 001 107
- SU-A- 1 622 357
- ZHANG R H ET AL: "Anaerobic treatment of swine waste by the anaerobic sequencing batch reactor" TRANS ASAE;TRANSACTIONS OF THE ASAE MAY-JUN 1997 ASAE, ST. JOSEPH, MI, USA, Bd. 40, Nr. 3, Mai 1997 (1997-05), Seiten 761-767, XP009019011
- O'KEEFE D M ET AL: "Influence of methane enrichment by aeration of recirculated supernatant on microbial activities during anaerobic digestion" BIORESOURC TECHNOL;BIORESOURCE TECHNOLOGY 2000 ELSEVIER SCIENCE LTD, EXETER, ENGL, Bd. 71, Nr. 3, 2000, Seiten 217-224, XP002258267
- STEPHENSON R J ET AL: "Effects of oxygenation and upflow liquid velocity on a coupled anaerobic/aerobic reactor system" WATER RESEARCH, ELSEVIER SCIENCE PUBLISHERS, AMSTERDAM, NL, Bd. 33, Nr. 12, August 1999 (1999-08), Seiten 2855-2863, XP004174053 ISSN: 0043-1354

## Beschreibung

Die Erfindung betrifft ein Verfahren zur Inbetriebnahme von methanhaltige Gase erzeugenden oder führenden Einrichtungen, insbesondere Biogasanlagen oder biogasführenden Leitungen, wobei in den Einrichtungen befindlicher Sauerstoff durch Anreicherung mit dem methanhaltigen Gas aus den Einrichtungen verdrängt und an die Atmosphäre abgegeben wird.

Bei der Erzeugung oder Weiterbehandlung von methanhaltigen Gasen, z. B. Biogas, Deponiegas oder Klärgas, werden an die Inbetriebnahme der zugehörigen Einrichtungen hohe Ansprüche gestellt. Insbesondere beim Anfahren von Biogasanlagen erfordern verfahrenstechnische und sicherheitstechnische Abhängigkeiten der Teilschritte der Inbetriebnahme besondere Aufmerksamkeit. Auch aufgrund hoher Sensibilität beim Umgang mit Impfmaterial ist ein zügiger, möglichst unterbrechungsfreier Ablauf von großer Bedeutung.

Während der Anfahrphase entsteht mit Einsetzen der Methanproduktion ein Methan-Luft-Gemisch, welches unter Verfügbarkeit einer externen Zündquelle explodieren kann. Um Gefahren bei der Inbetriebnahme von Biogasanlagen zu vermeiden, hat man deshalb bisher den Biogasreaktor und ggf. biogasführende Leitungen des nachgeschalteten Biogassystems mit Stickstoff inertisiert. Die Inbetriebnahme wies dabei folgende Teilschritte auf: -

Zunächst wurde der Biogasreaktor zur Vorwärmung mit Wasser befüllt. Anschließend wurde Stickstoff in den Biogasreaktor eingeleitet, wodurch der Reaktorinhalt inertisiert wurde. Nach dem Entleeren wurde dieser mit biogaserzeugenden. Mikroorganismen geimpft. Nach Zugabe von zu vergärendem Substrat wurde schließlich die Biogasproduktion gestartet.

Auch biogasführende Leitungen und Einrichtungen des dem Biogasreaktor nachgeschalteten Biogassystems zur Weiterbehandlung des Im Biogasreaktor erzeugten Biogases wurden bisher vor dem Betrieb mit Stickstoff inertisiert, um während der Anfahrphase mit einsetzender Methanproduktion die Bildung eines explosionsfähigen Methan-Luft-Gemisches zu verhindern.

Die Inertisierung mit Stickstoff ist jedoch mit Nachteilen verbunden:

Eine vollständige Inertisierung von insbesondere größeren Biogasreaktoren mit z. B. mehr als 6000 m³ Reaktorvolumen ist auch mit großen Stickstoffmengen nicht möglich, da sich im zu inertisierenden Biogasreaktor zwangsläufig sogenannte Totzonen bilden, in denen unerwünschte Restsauerstoffgehalte verbleiben. Außerdem ist der Inertisierungsvorgang sehr langwierig, was aufgrund des erhöhten Zeit- und Personalaufwarids wirtschaftliche Nachteile verursacht. Darüberhinaus ist mit erhöhten Kosten durch den sehr hohen Stickstoffeinsatz zu rechnen.

Als Alternative ist auch schon vorgeschlagen worden, die Luft aus gasführenden Leitungen mit Hilfe eines Kolbens mechanisch zu verdrängen.

Die Aufgabe der vorliegenden Erfindung besteht in der Bereitstellung eines alternativen Inertisierungsverfahrens bei Inbetriebnahne von Methan bzw. Biogas erzeugenden Anlagen.

Diese Aufgabe wird erfindungsgemäß dadurch gelöst, dass

das Verdrängen des Sauerstoffs solange aufrechterhalten wird, bis in dem an die Atmosphäre abgegebenen Gasgemsich eine Sauerstoffkonzentration von 6 Volumenprozent unterschritten wird. Die Einrichtungen gelten dann als inertisiert, sodass keine Explosionsgefahr mehr besteht und der Normalbetrieb aufgenommen werden kann.

Die Erfindung beruht dabei auf dem Prinzip, den Luftsauerstoff durch Anreichern mit Methan zu verdrängen. Das entstehende Methan-Luft-Gemisch wird kontrolliert an die Atmosphäre abgelassen. Aufgrund des Dichteunterschieds von Methan und Luft reduziert sich die Explosionsgefahr schnell mit zunehmender Entfernung des Ablasses vom.Erdboden.

Bei der Inbetriebnahme von Biogasreaktoren zeigen sich die Vorteile des erfindungsgemäßen Verfahrens gegenüber der herkömmlichen Inertisierung mit Stickstoff besonders deutlich. Da die Biogasbildung gleichmäßig über der gesamten

Aus DE 37 37 870 A, DE 100 01 107 A, ZHANG R H ET AL: "Anaerobic treatment of swine waste by the anaerobic sequencing batch reactor" TRANS ASEA; TRANSACTIONS OF THE ASAE MAY-JUN 1997 ASAE, ST. JOSEPH, MI, USA, Bd. 40, Nn 3, Mai 1997 (1997-05), Seiten 761-767 KP 009019011 , und O'KEEFE DM ET AL: "Influence of methane enrichment by aeration of recirculated supernatant on microbial activities during anaerobic digestion" BIORESOURC TECHNOL ; BIORESOURCE TECHNOLOGY 2000, EUSEVIER SCIENCE LTD ; EXETER, ENGL, Bd. 71, Nr. 3, 2000, Seiten 217 - 224, XP 002258267 sind Verfahren zur INbetriebnahme von methangaserzeugenden Einrichtungen bekannt, die die Verdrängung von Sauerstoff durch Anreicherung mit methanhaltigem Gas, sowie Algabe an die Atmosphäre umfassen.

Die SU-A-1 622 357 offenbart ein Verfahren zur Inbetriebnahme von Biogasanlagen ; das die Messung einer Sauerstoffkonzentrationsschwelle zur Explosionsvermeidung umfasst. Dabei wird Inertgas verwendet, welches erst bei Unterschreiten einer gewissen Sauerstoffkonzentration (2,5%) nicht mehr eïngeleitet wird.

In der DD 228 535 ist schließlich ein Verfahren zum Abfahren von Biogasanlagen bei gleichzeitiger Inertisierung beschrieben. Dabei wird Luft direkt in den Faulschlamm eingeblasen. Durch die Empfindlichkeit der methanproduzierenden Bakterien genenüber Sauerstoff sinkt der Methangehalt des Biogases, dessen Produktion allmählich zum Erliegen kommt. Die Lufteinblasung erfolgt derart, dass der Sauerstoffgehalt des gebildeten Gasgemisches eine maximale konzentration von 6% nicht übersteigt. Bei einem Methangehalt von kleiner 2% ist der Inertisierungsprozess beendet und der Biogasreaktor kann gefahrlos geöffnet werden.

Gärgutoberfläche erfolgt, treten keine Totzonen mit erhöhter Sauerstoffkonzentration auf, wie dies bei der Stickstoffinertisierung häufig vorkommt.

Zweckmäßigerweise wird bei der Inbetriebnahme des Biogasreaktors zunächst der Gasweg zu nachgeschalteten Einrichtungen zur Weiterbehandlung des Biogases abgesperrt und mindestens eine Ausblasöffnung im oberen Berreich des Biogasreaktors geöffnet. Der Biogasreaktor wird dann mit Wasser befüllt, welches beispielsweise mittels eines Wärmetauschers und einer Kreislaufpumpe auf z. B. 35°C erwärmt wird. Anschließend wird eine Impfung mit biogaserzeugenden Mikroorganismen, z. B, mit Klärschlamm aus einer anaerob-mesophilen Stufe einer Kläranlage, durchgeführt. Nach Zugabe des zu vergärenden Substrats wird schließlich die Biogasproduktion gestartet. Die Sauerstoffkonzentration im durch die Ausblasöffnung abgelassenen Gasgemisch wird vorzugsweise kontinuierlich gemessen und bei Unterschreitung einer Sauerstoffkonzentration von 6 Volumenprozent wird die Ausblasöffnung geschlossen und der Gasweg zu den nachgeschalteten Einrichtungen zur Weiterbehandlung des Biogases geöffnet. Nun kann der Normalbetrieb beginnen.

Die Erfindung kann zur sicheren Inbetriebnahme aller denkbaren Einrichtungen eingesetzt werden, die methanhaltige Gase erzeugen oder führen. Insbesondere ist die Erfindung für die Inbetriebnahme von Biogasanlagen gedacht, wobei sowohl der Biogasreaktor also auch biogasführende Leitungen des gesamten für die Biogasgewinnung und- aufbereitung vorgesehenen Biogassystems.auf die beschriebene Weise inbetriebgenommen werden können.

Von Vorteil ist beim erfindungsgemäßen Verfahren insbesondere, dass eine vollständige Sauerstoffevakuierung ohne Totzonen auch bei größeren Reaktorvolumina ermöglicht wird. Außerdem ist der Zeit- und Personalaufwand gegenüber der herkömmlichen Stickstoffinertisierung Wesentlich reduziert. Zusätzliche Kosten für Stickstoff fallen nicht an.

Im Folgenden soll die Erfindung anhand von Ausführungsbeispielen näher erläutert werden:

Das erste Ausführungsbeispiel betrifft die inbetriebnahme eines Biogasreaktors :

Im vorliegenden Ausführungsbeispiel handelt es sich um einen herkömmlichen Biogasreaktor zur Erzeugung von methanhaltigem Biogas aus zu vergärendem Substrat, z. B. Bioabfällen. Zur Inbetriebnahme des Bioreaktors wird zunächst der Gasweg zum nachgeschalteten Biogassystem abgesperrt. Es wird eine Atmung des Bioreaktors über eine Öffnung im Dom gewährleistet Auf dem Dach des Bioreaktors stehen in ca. 25m Höhe verschiedene Ausblasöffnungen zur Verfügung. Zur Vorwärmung des Bioreaktors wird ca. 3000m³ Wasser in den Bioreaktor eingefüllt. Das Wasser wird über Wärmetauscher und eine Kreislaufpumpe auf eine Temperatur von ca. 35°C aufgewärmt. Anschließend wird der Biogasreaktor mit Klärschlamm aus einer anaerob-mesophilen Stufe einer Kläranlage beimpft Dabei wird Impfmaterial in einer Menge von ca. 1500m³ zugegeben. Als zu vergärendes Substrat wird ca. 2000m³ dünne Maische aus Maischepuffer,in den Biogasreaktor eingefüllt. Die Gaszusammensetzung im Inneren des Biogasreaktors wird ab dem Impfzeitpunkt überwacht. Sobald eine Methanbildung und eine Sauerstoffzehrung nachweisbar sind, wird die dosierte Maischemenge stetig erhöht. Dabei werden ph-Wert und Temperatur beobachtet. Die erzeugte Biogasmenge verdrängt die im Gasraum des Biogasreaktors befindliche Luft und strömt über die Ausblasöffnung auf dem Dach des Biogasreaktors in die Atmosphäre. Das austretende Biogas ist leichter als Luft und steigt nach oben. Die Sauerstoffevakuierung gilt als abgeschlossen, wenn die Sauerstoffkonzentration im austretenden Gas auf weniger als 4 Volumenprozent gesunken ist. Sobald dieser Wert unterschritten ist, wird die Ausblasöffnung geschlossen und der Gasweg zum nachgeschalteten Biogassystem geöffnet. Der Normalbetrieb kann nun beginnen.

Das zweite Ausführungsbeispiel betrifft die Inbetriebnahme von Biogasrohrleitungen:

Bei der Inbetriebnahme des Biogassystems werden die einzelnen Biogasleitungen mit Biogas durchgeblasen. Dabei werden einerseits der Anschluss zum bereits biogasführenden System und anderseits eine Ausblasöffnung am anderen Ende der Biogasleitung geöffnet. Das aus dem Biogasreaktor kommende Biogas verdrängt die Luft aus der Biogasrohrleitung und strömt über die Ausblasöffnung an sicherer Stelle in die Atmosphäre. Das austretende Biogas ist leichter als Luft und steigt nach oben. Die Spülung der Biogasrohrleitung erfolgt bei maximalem Gasdruck im Biogasreaktor von z. B. 55 Millibar. Es werden lediglich kurze Spülzeiten vorgenommen, sodass der Druck im Gasraum des Bioreaktors während der Spülung unverändert bleibt. Die notwendige Höhe der Ausblasöffnung wird unter Beachtung der örtlichen, technologischen und sicherheitstechnischen Aspekte festgelegt: Im vorliegenden Ausführungsbeispiel ist eine Höhe von 10m vorgesehen.

## Patentansprüche

1. Verfahren zur Inbetriebnahme von methanhaltige Gase erzeugenden oder führenden Einrichtungen insbesondere Biogasanlagen oder biogasführenden Leitungen, wobei in den Einrichtungen befindlicher Sauerstoff durch Anreicherung mit dem methanhaltigen Gas aus den Einrichtungen verdrängt und an die Atmosphäre abgegeben wird,
**dadurch gekennzeichnet, dass** das Verdrängen des Sauerstoffs solange aufrechterhalten wird, bis in dem an die Atmosphäre abgegeben Gasgemisch eine Sauerstoffkonzentration von 6 Volumenprozent unterschritten wird.

2. Verfahren nach Anspruch 1, **dadurch gekennzeichnet, dass** zur Inbetriebnahme eines Biogasreaktors zunächst der Gasweg zu nachgeschalteten Einrichtungen zur Weiterbehandlung des Biogases abgesperrt wird und mindestens eine Ausblasöffnung im oberen Bereich des Biogasreaktors geöffnet wird, der Biogasreaktor z. B. durch Befüllen mit Wasser vorgewärmt wird, anschließend eine Impfung mit biogaserzeugenden Mikroorganismen durchgeführt wird und zu vergärendes Substrat hinzugeführt wird, die Sauerstoffkonzentration im durch die Ausblasöffnung abgelassenen Gasgemisch gemessen wird und bei Unterschreiten einer Sauerstoffkonzentration von 6 Volumenprozent die Ausblasöffnung geschlossen und der Gasweg zu den nachgeschalteten Einrichtungen zur Weiterbehandlung des Biogases geöffnet wird.

## Claims

1. Method for starting up facilities generating or carrying gases containing methane, in particular biogas plants or biogas-carrying lines, wherein oxygen existing in the facilities is displaced from the facilities by enrichment with the methane-containing gas and discharged to the atmosphere, **characterized in that** the displacement of the oxygen is maintained until the oxygen content in the gas mixture discharged to the atmosphere falls below 6 per cent by volume.

2. Method according to Claim 1, **characterized in that** for starting up a biogas reactor, firstly, the gas path to downstream facilities for further treatment of the biogas is closed off and at least one blow-out opening in the upper portion of the biogas reactor is opened, the biogas reactor is preheated, for example by filling with water, inoculation with biogas-generating microorganisms is then carried out and substrate to be fermented is introduced, the oxygen concentration in the gas mixture discharged through the blow-out opening is measured and, when the oxygen concentration falls below 6 per cent by volume, the blow-out opening is closed and the gas path to the downstream facilities for further treatment of the biogas is opened.

## Revendications

1. Procédé en vue de la mise en service d'équipements produisant ou conduisant des gaz contenant du méthane, en particulier d'installations de biogaz ou de conduits transportant des biogaz, l'oxygène se trouvant dans les équipements étant expulsé par enrichissement à l'aide d'un gaz contenant du méthane et étant évacué à l'atmosphère, **caractérisé en ce que** l'expulsion de l'oxygène est maintenue active jusqu'à que l'on ce passe en dessous d'une concentration d'oxygène de 6 pour cent en volume dans le mélange gazeux évacué à l'atmosphère.

2. Procédé selon la revendication 2, **caractérisé en ce qu'**en vue de la mise en service d'un réacteur à biogaz, on bloque tout d'abord le trajet de gaz en direction des équipements montés en aval en vue du traitement ultérieur du biogaz et **en ce que** l'on ouvre au moins une ouverture d'évacuation dans le domaine supérieur du réacteur à biogaz, **en ce que** l'on procède au chauffage préalable du réacteur à biogaz, par exemple, par remplissage à l'aide d'eau, **en ce que** l'on effectue à la suite une inoculation à l'aide de microorganismes produisant du biogaz et **en ce que** l'on introduit le substrat en cours de fermentation, **en ce que** l'on mesure la concentration d'oxygène dans le mélange gazeux évacué à l'aide de l'ouverture d'évacuation et **en ce qu'**au cas où l'on passe en dessous d'une concentration en oxygène de 6 pour cent en volume, l'on ferme l'ouverture d'évacuation et **en ce que** l'on ouvre le trajet de gaz en direction des équipements montés en aval en vue du traitement ultérieur du biogaz.
